# EUROPEAN PATENT APPLICATION

(11) **EP 1 092 783 A1**
(43) Date of publication of application: **18.04.2001**
(21) Application number: 98938949.9
(22) Date of filing: 24.08.1998
(51) Int. Cl.: C12Q 1/68

(54) **METHOD OF CANCER DIAGNOSIS BASED ON THE DETECTION OF CHROMOSOME ABNORMALITY**

(30) Priority: 25.02.1998 JP 4355198; 17.06.1998 JP 16952298
(71) Applicant: Sumitomo Electric Industries, Ltd., Osaka-shi, Osaka 541-0041 (JP)
(72) Inventor: KISHIMOTO, Toshihiko, Yokohama Works, Yokohama-shi Kanagawa 244-8588 (JP); NIWA, Shin-ichiro Yokohama Works, Yokohama-shi Kanagawa 244-8588 (JP); SASAKI, Kohsuke Yamaguchi University, Ube-shi Yamaguchi 755-8505 (JP)
(74) Representative: Baldock, Sharon Claire
(86) International application number: JP9803733
(87) International publication number: WO9943196

(57) **Abstract**

It has now been revealed that, in the chromosomes of cells of meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma or oral squamous cell carcinoma, there is amplifications or deletions of DNA in the regions specific for each cancer. The present invention provides a method for diagnosing cancers by detecting the amplification or deletion in chromosome regions which are specific for each of the above cancers.

## Description

### Technical Field

The present invention relates to a method for diagnosing meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma and oral squamous cell carcinoma. More specifically, the present invention relates to a method for diagnosing these cancers by detecting amplification or deletion of a specific region of chromosome derived from a patient.

### Background Art

Cancer is a disease where some cells abnormally grow and invade into surrounding normal tissues to destroy their normal functions. Since 1981, cancer is the primary factor of death in Japan, and it is socially necessary to rapidly establish a method for effectively diagnosing and treating the disease.

Up to date, a variety of research on cancer have been made in gene level. As a result, it has been revealed that deletion or amplification of a part of chromosome correlates to carcinogenesis. For example, it has been found that Rb gene of human chromosome 13 is always deficient in retinoblastoma, and it has been found that p53 gene of human chromosome 17 short arm is deficient in a variety of cancers. These genes are tumor suppressor genes and it is thought that deletion of these genes causes carcinogenesis. Further, it is found that amplification of a specific region of the chromosome occurs in certain cancers. Recently, a theory is proposed that cancer arises from multi-step mutations of deletion and/or amplification of not one but several genes in genome.

As mentioned above, the relationship between carcinogenesis and chromosome aberration has been actively studied, but the relationship has not been fully revealed and mechanism of caracinogenesis has not yet been revealed. Particularly, although there are a large number of patients with meningioma, glioma, biliary region carcinoma, ovarian clear cell adenocarcinoma, non-clear cell adenocarcinoma, corpus uteri cancer, hepatocellular carcinoma, pituitary adenoma, stomach cancer, esophageal squamous cell carcinoma or oral squamous cell carcinoma, structural changes of chromosomes caused by these cancers have not been clarified yet.

### Summary of the Invention

One object to be achieved by the present invention is to reveal a specific structural change of chromosomes in meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma or oral squamous cell carcinoma. Another object to be achieved by the present invention is to provide a method for diagnosing these cancers by detecting a specific structural change of chromosomes in meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma or oral squamous cell carcinoma. Still, another object of the present invention is to provide a method with which these diagnoses can be simply carried out in a single procedure.

In order to achieve the above-mentioned objects, the present inventors have analyzed DNA of each cancer cell of meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma or oral squamous cell carcinoma using CGH (comparative genomic hybridization) method. As a result, it has been found that there is amplification or deletion of DNA in the regions which are specific for each cancer in the chromosomes of each cancer and that it is possible to correlate the structural change of chromosomes and the onset of a cancer. On the basis of this fact, the present inventors have developed a method for diagnosing each cancer by detecting cancer-specific amplification or deletion, and consequently accomplished the present invention.

In the first aspect of the present invention, there are provided: a method for diagnosing benign meningioma, which comprises a step of detecting deletion in human chromosome 22 long arm in the chromosomes of cancer cells derived from a patient; a method for diagnosing atypical meningioma, which comprises a step of detecting deletion in human chromosome 1 short arm, human chromosome 6, human chromosome 14 long arm, human chromosome 18, or human chromosome 22 long arm in the chromosomes of cancer cells derived from a patient; and a method for diagnosing anaplastic meningioma, which comprises a step of detecting amplification in human chromosome 20 long arm, or detecting deletion in human chromosome 1 short arm, human chromosome 2 short arm, human chromosome 6 long arm, human chromosome 10, human chromosome 14 long arm or human chromosome 22 long arm, in the chromosomes of cancer cells derived from a patient.

In the second aspect of the present invention, there are provided: a method for diagnosing malignant glioma, which comprises a step of detecting amplification in human chromosome 7 short arm, human chromosome 7 long arm, human chromosome 20 long arm or human chromosome 8 long arm, or detecting deletion in human chromosome 10 short arm, human chromosome 9 short arm, human chromosome 10 long arm, human chromosome 13 long arm or human chromosome 22 long arm in the chromosomes of cancer cells derived from a patient; a method for diagnosing primary malignant glioma, which comprises a step of detecting amplification in human chromosome 7 short arm, human chromosome 7 long arm or human chromosome 20 long arm, or detecting deletion in human chromosome 10 or human chromosome 22 long arm in the chromosomes of cancer cells derived from a patient; and a method for diagnosing metastatic malignant glioma, which comprises a step of detecting amplification in human chromosome 8 long arm or human chromosome 10 short arm, or detecting deletion in human chromosome 17 short arm in the chromosomes of cancer cells derived from a patient.

In the third aspect of the present invention, there are provided: a method for diagnosing biliary region carcinoma, which comprises a step of detecting amplification in human chromosome 17 long arm, human chromosome 19 long arm, human chromosome 12 short arm, human chromosome 5 short arm, human chromosome 8 long arm, human chromosome 1 long arm, human chromosome 7 short arm, human chromosome 20 long arm or human chromosome X long arm, or detecting deletion in human chromosome 6 long arm, human chromosome 4 long arm, human chromosome 5 long arm, human chromosome 9 short arm, human chromosome 13 long arm or human chromosome 18 long arm in the chromosomes of cancer cells derived from a patient; and a method for diagnosing anomalous confluence of the pancreaticobiliary ductal system in gallbladder cancer, which comprises a step of detecting amplification or deletion in human chromosome 18 long arm in the chromosomes of cancer cells derived from a patient.

In the forth aspect of the present invention, there is provided: a method for diagnosing ovarian clear cell adenocarcinoma, which comprises a step of detecting amplification in human chromosome 8 long arm, human chromosome 17 long arm, human chromosome 20 long arm, human chromosome 21 long arm or human chromosome 22 long arm, or detecting deletion in human chromosome 6 long arm, human chromosome 8 short arm, human chromosome 10 short arm, human chromosome 17 short arm, or human chromosome 19 in the chromosomes of cancer cells derived from a patient.

In the fifth aspect of the present invention, there is provided a method for diagnosing non-clear cell adenocarcinoma, which comprises a step of detecting amplification in human chromosome 3 long arm in the chromosomes of cancer cells derived from a patient, is provided.

In the sixth aspect of the present invention, there is provided: a method for diagnosing corpus uteri cancer, which comprises a step of detecting amplification in human chromosome 3 long arm, human chromosome 8 long arm, human chromosome 4 long arm, human chromosome 13 long arm or human chromosome X long arm, or detecting deletion in human chromosome 1 short arm, human chromosome 16 short arm, human chromosome 17 short arm, human chromosome 19 long arm, human chromosome 15 long arm or human chromosome 22 long arm in the chromosomes of cancer cells derived from a patient.

In the seventh aspect of the present invention, there are provided: a method for diagnosing hepatocellular carcinoma, which comprises a step of detecting amplification in human chromosome 1 long arm, human chromosome 8 long arm, human chromosome 19 long arm or human chromosome 20 long arm, or detecting deletion in human chromosome 8 short arm, human chromosome 13 long arm, human chromosome 16 long arm or human chromosome 17 short arm in the chromosomes of cancer cells derived from a patient; a method for diagnosing hepatitis C virus-positive hepatoma, which comprises a step of detecting deletion in human chromosome 16 long arm or human chromosome 10 long arm in the chromosomes of cancer cells derived from a patient; and a method for diagnosing hepatitis B virus-positive hepatoma, which comprises a step of detecting amplification at human chromosome 11 long arm 13, in the chromosomes of cancer cells derived from a patient.

In the eighth aspect of the present invention, there is provided a method for diagnosing pituitary adenoma, which comprises a step of detecting deletion in human chromosome 13 long arm in the chromosomes of cancer cells derived from a patient.

In the ninth aspect of the present invention, there is provided: a method for diagnosing stomach cancer, which comprises a step of detecting amplification in human chromosome 8 long arm, human chromosome 3 long arm or human chromosome 20 long arm, or detecting deletion in human chromosome 17 short arm, human chromosome 19 short arm or human chromosome 19 long arm in the chromosomes of cancer cells derived from a patient.

In the tenth aspect of the present invention, there is provided: a method for diagnosing esophageal squamous cell carcinoma, which comprises a step of detecting amplification in human chromosome 3 long arm, human chromosome 11 long arm, human chromosome 8 long arm or human chromosome 5 short arm, or detecting deletion in human chromosome 3 short arm, human chromosome 4 long arm, human chromosome 9 short arm or human chromosome 18 long arm in the chromosomes of cancer cells derived from a patient.

In the eleventh aspect of the present invention, there is provided: a method for diagnosing oral squamous cell carcinoma which comprises a step of detecting amplification in human chromosome 5 short arm, human chromosome 8 long arm, human chromosome 20 short arm, human chromosome 20 long arm or human chromosome 3 long arm, or detecting deletion in human chromosome 18 long arm or human chromosome 4 long arm in the chromosomes of cancer cells derived from a patient.

As one embodiment of carrying out the method for diagnosis provided by the first to eleventh aspects of the present invention, the method for diagnosis is provided, wherein said amplification or said deletion is detected by:
competitively hybridizing DNA of cancer cells derived from a patient which is labeled with a first label and DNA of normal cells which is labeled with a second label, to chromosomes of normal cells; and
observing a chromosome region to which DNA of cancer cells is hybridized by the first label, observing a chromosome region to which DNA of normal cells is hybridized by the second label, observing a chromosome region to which both DNAs are hybridized by a mixture of the first and second labels, and distinguishing the labels in each region of the chromosome.

As another embodiment of carrying out the method for diagnosis provided by the first to eleventh aspects of the present invention, the method for diagnosis is provided, wherein said amplification or said deletion is detected by:
competitively hybridizing DNA of cancer cells derived from a patient which is labeled to be detected as a first color and DNA of normal cells which is labeled to be detected as a second color, to chromosomes of normal cells; and
observing a chromosome region to which DNA of cancer cells is hybridized by the first color, observing a chromosome region to which DNA of normal cells is hybridized by the second color, observing a chromosome region to which both DNAs are hybridized by the third color formed by a mixture of the first and second colors, and distinguishing the colors in each region of the chromosome. In this embodiment, amplification or deletion can be detected by measuring the ratio of intensity between the first and second colors in each region of the chromosomes.

A method for diagnosing each tumor or cancer by detecting amplification or deletion of genes contained in the chromosome regions which have been revealed in the present invention, is within the method for diagnosis of the present invention

### Brief Description of the Drawings

Figure 1 shows the regions of human chromosome which are found to be amplified in meningioma.
Figure 2 shows the regions of human chromosome which are found to be deleted in meningioma.
Figure 3 shows the regions of human chromosome which are found to be amplified or deleted in biliary region carcinoma.
Figure 4 shows the number of genomic aberrations in each stage of biliary region carcinoma.
Figure 5 shows the regions of human chromosome which are found to be amplified or deleted in ovarian clear cell adenocarcinoma.
Figure 6 shows the regions of human chromosome which are found to be amplified or deleted in corpus uteri cancer.
Figure 7 shows the regions of human chromosome which are found to be amplified or deleted in hepatocellular carcinoma.
Figure 8 shows the regions of human chromosome which are found to be amplified or deleted in pituitary adenoma.
Figure 9 shows the regions of human chromosome which are found to be amplified or deleted in stomach cancer.
Figure 10 shows the regions of human chromosome which are found to be amplified or deleted in esophageal squamous cell carcinoma.

### Preferred Embodiments for Carrying out the Invention

The method for carrying out the invention is described in detail below.

The method for diagnosing meningioma, glioma, pituitary adenoma, biliary region carcinoma, ovarian clear cell adenocarcinoma, non-clear cell adenocarcinoina, corpus uteri cancer, hepatocellular carcinoma, stomach cancer, esophageal squamous cell carcinoma or oral squamous cell carcinoma according to the present invention is characterized in that aberration of chromosome regions specific for each cancer is detected. Therefore, the diagnosis of the present invention may be carried out by any means which is capable of detecting change of chromosome regions, aberration of which is intended to be detected. As the method of diagnosing corpus uteri cancer of the present invention, for example, any means may be used, as long as it can detect amplification in human chromosome 3 long arm, human chromosome 8 long arm, human chromosome 4 long arm, human chromosome 13 long arm or human chromosome X long arm, or can detect deletion in human chromosome 1 short arm, human chromosome 16 short arm, human chromosome 17 short arm, human chromosome 19 long arm, human chromosome 15 long arm or human chromosome 22 long arm in the chromosomes of cancer cells derived from a patient

As such a method for detecting chromosomal aberration, *in situ* hybridization method may be used. The *in situ* hybridization method is a method where a labeled nucleic acid probe is hybridized with DNA of chromosomes, and then the label is detected, whereby the chromosome region to which the nucleic acid probe was hybridized is identified.

By using this method, the probe which is prepared by labeling genomic DNA obtained from cells of tumor tissues from patients can be hybridized to chromosomes of normal cells. The chromosome region of normal cells to which the probe does not hybridize represents a region which is deleted in the chromosomes of cancer cells. Further, the probe which is prepared by labeling genomic DNA obtained from normal cells can be hybridized to chromosomes prepared from tumor tissues from a patient. The chromosome region of cancer cells to which the probe does not hybridize represents a region which is amplified in the chromosomes of cancer cells. As mentioned above, in the case where either one of deletion or amplification of chromosomes of cancer cells is detected, *in situ* hybridization method can be effectively applied.

When it is necessary to simultaneously detect deletion and amplification of chromosomes of cancer cells in a single procedure, CGH method is preferably used. Particularly, in each method for diagnosis of anaplastic meningioma, glioma, anomalous confluence of the pancreaticobiliary ductal system in gallbladder cancer, ovarian clear cell adenocarcinoma, corpus uteri cancer, hepatocellular carcinoma, stomach cancer or oral squamous cell carcinoma according to the present invention, CGH method is preferably used.

In CGH method, DNA labeled with a first label and another DNA labeled with a second label are simultaneously hybridized to chromosomes, and the chromosome region to which each DNA is hybridized is identified by detecting the 2 labels respectively (Du Manoir, S. *et al., Hum. Genet.,* 90:590-610, 1993). In the method of diagnosis of the present invention, DNA probe prepared from DNA of cancer cells from a patient and labeled with a first label and DNA probe prepared from DNA of normal cells and labeled with a second label are simultaneously hybridized to chromosomes of normal cells. In the regions amplified in cancer cells among the chromosomes, the first label is observed since DNA of cancer cells are dominantly hybridized. On the other hand, in the regions deleted in cancer cells, the second label is observed since only DNA of normal cells are hybridized. In the consensus regions unchanged in cancer cells and normal cells, the first and second labels are observed as a mixed color since both DNAs are competitively hybridized. Thus, deletion and amplification of chromosomes in cancer can be detected in a single procedure.

The following is a specific description on the method of diagnosis of the present invention using CGH method which is a preferred aspect of the present invention.

DNA of cancer cells used in the method for diagnosis of the present invention is prepared from the cells collected from tumor tissues of a patient with suspected meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma or oral squamous cell carcinoma. The terms "cancer cells" and "tumor tissues" of a patient used herein include cells and tissues which have been already in cancerous state as well as those which are in a precancerous state.

The term "meningioma" used herein has a broad sense, including benign meningioma, atypical meningioma and anaplastic meningioma. The term "glioma" used herein has a broad sense, including malignant glioma, primary malignant glioma and metastatic malignant glioma. The term "biliary region carcinoma" has a broad sense, including gallbladder cancer, intrahepatic cholangioma, hepatic portal cholangiomas, extrahepatic cholangiomas and papilloma, and further gallbladder cancer includes the one with anomalous confluence of the pancreaticobiliary ductal system. Furthermore, the term "hepatocellular carcinoma" has a sense which includes both hepatitis C virus-positive hepatoma and hepatitis B virus-positive hepatoma.

DNA of normal cells used in the method for diagnosis of the present invention is DNA of cells derived from a healthy person having no cancer. The type of tissues or cells to be collected is not particularly limited.

As long as DNA of cancer cells and DNA of normal cells to be prepared contain the segments corresponding to the chromosome region, of which deletion or amplification is to be detected, the method for diagnosis of the present invention can be carried out without any problem. However, generally genomic DNA is used in order to avoid complexity of selecting DNA of a specific chromosome region.

Genomic DNA can be prepared from the collected cells by using a suitable restriction enzymes according to a method known in the art. Since the length of each DNA used in hybridization as a probe is 200 ― 1000 bp, the length of the genomic DNA is preferably within the range of 10 ― 1000 kb. However, DNA having a length other than this range may be used.

DNA of cancer cells and DNA of normal cells are respectively labeled with different labels (a first label and a second label) before hybridization. The type of the labels is not particularly limited as long as each label can be detected distinguishably with each other after hybridization. Therefore, spectroscopically, biochemically, chemically or immunologically detectable labels can be broadly used. For example, it is possible to use fluorescent dyes, electron density reagents, enzymes, biotin, digoxygenin and the like.

A preferred label is the one which can be visually recognized due to its color-development. *Inter alia*, it is preferred that a combination of labels is selected in such a way that the color of a first label and the color of a second label are mixed to form a third color which is distinguishable from these two colors. For example, by selecting a label which develops green color as a first label and a label which develops red color as a second label, yellow color can be observed when the two colors are mixed. Thus, by choosing labels in such a way that 3 colors which are distinguishable from one another are formed, and hybridizing DNA, regions deleted, amplified, or unchanged in cancer can be visually and easily distinguished.

Examples of the fluorescent dye of green which can be used in the present invention include FIITC (fluorescein isothiocyanate). Examples of the fluorescent dye of red include TRITC (tetramethylrhodamine isothiocyanate), rhodamine and Texas Red.

The condition under which DNA of cancer cells and DNA of normal cells are hybridized to chromosomes is not particularly limited. As chromosomes to which DNA of cancer cells and DNA of normal cells are hybridized, for example, chromosome samples prepared from cells such as lymphocytes can be used. Also, hybridization may be performed using unfixed chromosomes, and then the chromosomes may be dropped onto a slide glass or the like, and fixed.

The method of analysis after hybridization is appropriately determined depending on the type of labels. When fluorescent dye is used as a label, analysis can be performed by visually observing the sample using high resolution fluorescence microscopy and the like. Also, the image observed by the fluorescence microcopy can be read by a color image scanner and the colors at each point are resolved into 3 colors, and quantification can be performed as to which of the colors of green or red is major at each point (namely, ratio of labeling materials at each point can be determined).

The method of the present invention includes methods for diagnosing tumors or cancers by detecting amplification or deletion of genes contained in the chromosome regions which were identified by the present invention.

Thus, according to the present invention, by detecting deletion or amplification in the chromosomes of tissues derived from a patient, it is possible to easily diagnose whether or not the said tissues are affected by meningioma, glioma, biliary region carcinoma, ovarian clear cell adenocarcinoma, non-clear cell adenocarcinoma, corpus uteri cancer, hepatocellular carcinoma, pituitary adenoma, stomach cancer, esophageal squamous cell carcinoma or oral squamous cell carcinoma. The method of the present invention, therefore, is expected to expand the range of the possibilities of an early diagnosis or treatment of cancers.

### Examples

The present invention will be illustrated in more detail by the examples described below, but they should not be construed as limiting the scope of the invention.

### Example 1: Analysis of chromosomal aberration in meningioma

### (1) Preparation of chromosome sample

Using cells having normal chromosomes (blood cells from normal persons), chromosome samples were prepared by the following procedure.

10 ml of blood was collected from each person, and was diluted approximately 1.5 times with culture medium (RPMI 1640, 10% FCS, PHA(20 µl/ml)). The diluted blood was gently poured into a tube (Ficol Paque: Pharmacia), and centrifuged at 2,000 rpm for 20 minutes. The layer of leukocytes was taken out, diluted with the same medium, and centrifuged at 1,200 rpm for 5 minutes. The dilution and centrifugation procedures were repeated twice. The same culture medium was added to the leukocytes and well stirred, and then 15 ml of the mixture was placed in each of 2 dishes of 10 cm, and the culturing was started.

After the culture for 48 hours, thymidine (Sigma) was added to a final concentration of 300 µg/ml. After culture for 18 hours, the washing with the same medium accompanied by centrifugation at 1,200 rpm for 5 minutes was repeated three times. After culture for 6 hours, colcemid (Gibco) was added to a final concentration of 0.05 µg/ml. After 1 hour, the same medium was added to the cells, and the cells were washed by centrifugation at 1,200 rpm for 5 minutes and subjected to hypotonic treatment with 3-5 ml of 0.075M KCl for 30 minutes at room temperature. Carnoy's fixative (methanol: acetic acid = 3:1) was then added in several times until the color of erythrocytes disappeared, whereby the cells were fixed. The fixed cells were dropped onto slides and stored at -80°C until use.

### (2) Preparation of genomic DNA

Using tumor tissues from 13 patients with benign meningioma, 4 patients with atypical meningioma and 3 patients with anaplastic meningioma, genomic DNA was prepared by the following procedure.

The solid tumor tissue of 5 mm square or more was simply moistened with PBS and cut into small pieces with an ophthalmologic scissors. The cell suspension was charged into a syringe (1 ml) and discharged from it and this procedure was repeated several time, and then the cell suspension was filtered through nylon mesh. The cell suspension was poured into a centrifugation tube and 0.2% NaCl was added thereto and the tube was left for a few minutes. The suspension was centrifuged at 2,000 rpm for 5 minutes, and then the supernatant was discarded and the pellet was resuspended in a small amount of PBS. The cell suspension was placed in a microtube and centrifuged at 2,000 rpm for 5 minutes, and the supernatant was discarded. Then, DNA was prepared using the nucleic acid extracting reagent SEPAGENE™ (Sanko Juuyaku Inc.) in accordance with the manufacturer's instructions.

Also, genomic DNA for use as DNA of normal cells was prepared from lymphocytes of blood from a healthy person in accordance with the following procedure.

20 ml of blood with anti-coagulant, heparin, was collected from a healthy person, and was diluted twice with PBS. The diluted blood was gently layered on 30 ml Ficoll solution in a centrifugation tube, and left for 10 minutes. Then, the tube was centrifuged at 2,000 rpm for 20 minutes to form a milk white layer of lymphocytes under a pale yellow layer. The milk white layer was taken into another centrifugation tube containing 30 ml Ficoll solution and PBS was added thereto. The tube was centrifuged at 2,000 rpm for 5 minutes and the supernatant was removed by aspirator. 3 ml of 0.2% NaCl was added to cause hemolysis, and the sample was centrifuged at 2,000 rpm for 5 minutes, and the supernatant was removed by aspirator. Then, DNA was prepared using the nucleic acid extracting reagent SEPAGENE™ (Sanko Juuyaku Inc.) in accordance with the manufacturer's instructions.

### (3) Preparation of labeled DNA

DNA of meningioma cells was labeled with FITC which emits green fluorescence, and DNA of normal cells was labeled with TRITC which emits red fluorescence.

DNA was labeled by mixing dTTP, dNTP mixture, labeled d-UTP, template DNA, nick translation buffer, nick translation enzyme and water and incubating the mixture for an appropriate period of time in accordance with the instructions attached to Nick Translation Kit (Vysis).

### (4) Hybridization (FISH method)

### (A) Preparation of single-stranded DNA probe

Human COT-1 DNA (20 µl; 400ng of DNA), TRITC-labeled DNA of normal cells (10 µl; 200ng of DNA), FITC-labeled DNA of meningioma cells (10 µl; 200ng of DNA), sodium acetate (4 µl) and 100% ethanol (1ml) were mixed and centrifuged at 14,000 rpm for 30 minutes at 4°C. The supernatant was discarded by decantation and was completely dried in the dark. Then, 10 µl of Master Mix #1 was added and fully pipetted. Then, the mixture was treated at 75°C for 5 minutes to prepare single-stranded DNA.

### (B) Preparation of single-stranded chromosome sample

DNA of chromosome sample was treated with 70% formamide/2xSSC (pH7.0) for 2.5 minutes at 75°C to be single-stranded. The sample was then successively treated with 70% ethanol, 85% ethanol and 100% ethanol each for 2 minutes to be dehydrated, and was air-dried. By analyzing the location and number of bands on the chromosome, it was confirmed that the chromosome was normal one.

### (C) Hybridization

To a slide glass containing the chromosome sample was loaded 9.5 µl of the single-stranded probe-mix. The glass was covered with a cover glass of 18x18, and shielded with a paper bond. The glass was placed on a hot plate for about 10 minutes, and hybridization was performed in CO₂ incubator at 37°C for 2-3 days.

After hybridization, washing the sample with 50% formamide/2xSSC (pH7.0) at 45°C for 12 minutes was repeated three times. Then, washing the sample with 2xSSC at 45°C for 10 minutes and with PN buffer at room temperature for 10 minutes was repeated twice, and washing the sample with a distilled water at room temperature for 5 minutes was repeated twice. Then, 8 µl of 0.1-0.2 mg /ml DAPI-antifade was loaded to the sample, and the sample was a covered with a cover glass of 18x18.

### (5) Fluorescence microscopic examination

The slide glass was analyzed under fluorescence microscopy, and fluorescence microscopic photographs were taken (color photograph).

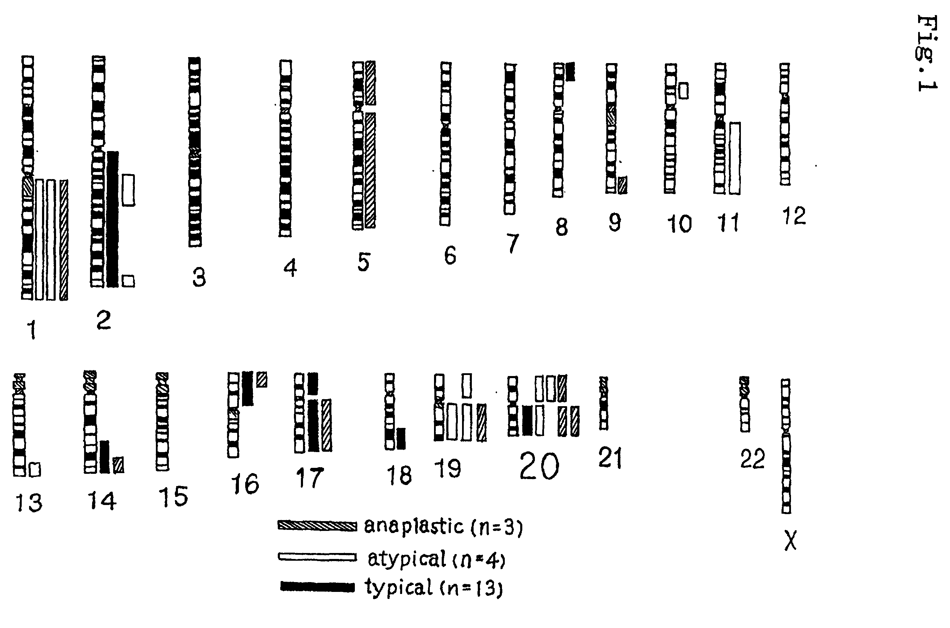

The regions in green in each chromosome, which correspond to regions indicating DNA amplification specific for meningioma, are shown in Figure 1 and Table 1. In Table 1, each number represents a chromosome number, p denotes the short arm, and q denotes the long arm. For example, 1p denotes human chromosome 1 short arm. The number expressed as a fraction in a parenthesis on the right of each chromosome region denotes the number of changed genes. The denominator denotes the number of analyzed genes and the numerator denotes the number of genes where a certain change appeared. In Figure 1, positions wherein the chromosomal amplification is shown are indicated by bars. One bar denotes that there is an increase of chromosome at the position in one patient.

**Table 1**

| | Positions of chromosomal amplification Specific for meningioma |
|---|---|
| Benign meningioma (n=13) | 2q (2/13), the top of 8p (1/13), |
| | the bottom of 14q (1/13), 16p (1/13), 17p (1/13), |
| | 17q (1/13), the bottom of 18q (1/13), 19q (1/13), |
| | 20 (1/13)q |
| Atypical meningioma (n=4) | 1q (2/4), the center of the upper part of 2q (1/4), |
| | the bottom of 2q (1/4), the center of 10p (1/4), |
| | 11q (1/4), the bottom of 13q (1/4), 19p (1/4), |
| | 19q (1/4), 20p (2/4), 20 (1/4)q |
| Anaplastic meningioma (n=3) | 1q (1/3), 5p (1/3), 5q (1/3), |
| | the bottom of 9q (1/3), the bottom of 14q (1/3), |
| | the top of 16p (1/3), 17q (1/3), 19q (1/3), |
| | 20p (1/3), 20q (2/3) |

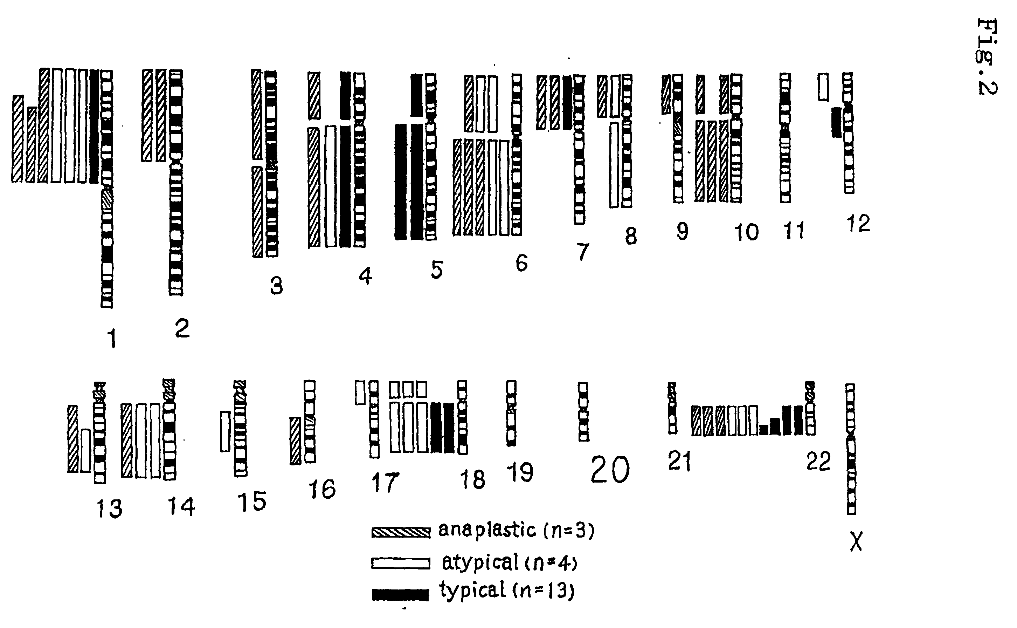

The regions in red in each chromosome, which correspond to regions indicating DNA deletion due to meningioma, are shown in Figure 2 and Table 2. In Figure 2, the positions wherein the deletion in a chromosome is shown are indicated by bars. One bar denotes that there is a deletion of chromosome at the position in one patient.

**Table 2**

| | Positions of chromosomal deletion in meningioma |
|---|---|
| Benign meningioma (n=13) | 1p (1/13), 4p (1/13), 4q (1/13), |
| | 5p (1/13), 5q (2/13), 7p (2/13), |
| | the top of 12q (1/13), 18q (2/13), |
| | 22q (2/13), the lower-half of 22q (1/13), |
| | the bottom of 22q (1/13) |
| Atypical meningioma (n=4) | 1p (3/4), 4q (1/4), 6p (2/4), 6q (3/4), |
| | 8p (1/4), 8q (1/4), 12p (1/4), |
| | the lower-center of 13q (1/4), 14q (2/4), |
| | the center of 15q (1/4), 17p (1/4), 18p (3/4), |
| | 18q (3/4), 22q (3/4) |
| Anaplastic meningioma (n=3) | 1p (1/3), 1p (except the top part) (2/3), |
| | 2p (2/3), 3p (1/3), 3q (1/3), 4p (1/3), |
| | 4q (1/3), 6p (1/3), 6q (2/3), 7p (1/3), |
| | 8p (1/3), 9p (1/3), 10p (2/3), 10q (3/3), |
| | 13q (except the top and bottom parts) (1/3), |
| | 14q (1/3), 16q (1/3), 22q (3/3) |

The above result suggests that the structural change of chromosome occurs with the progression of meningioma in the following order.

First, chromosome 22 long arm is deleted from chromosomes in cell of the cerebromeningeal layer to form benign meningioma. Second, chromosome 1 short arm, chromosome 6 long arm, chromosome 14 long arm and chromosome18 are further deleted to form atypical meningioma. Finally, the deletion in chromosome 10 and chromosome 2 short arm causes the change in structure of chromosome 20 long arm which is specific for meningioma, whereby anaplastic meningioma is formed.

### Example 2: Analysis of chromosomal aberration in malignant glioma

Using blood and tumor tissues from patients with malignant gliomas (33 patients), the same procedure as stated in Example 1 was carried out to prepare chromosome samples and DNA of cancer cells. Preparation of genomic DNA, labeling, hybridization and detection were carried out by the same method as in Example 1.

The results are shown in Table 3.

**Table 3**

| Chromosome regions with amplification (frequency) | Chromosome regions with deletion (frequency) |
|---|---|
| 7p13-p12 (10/33) | 10p12-p11 (13/33) |
| 7q31 (9/33) | 9p21 (12/33) |
| 20q13 (9/33) | 10q22-q ter (10/33) |
| 8q24 (8/33) | 13q21-q31 (8/33) |
| | 22q12-q13.2 (6/33) |

The above results indicate that the patients with malignant gliomas had the amplifications in chromosome 7 short arm, chromosome 7 long arm, chromosome 20 long arm and chromosome 8 long arm, and the deletions in chromosome 10 short arm, chromosome 9 short arm, chromosome 10 long arm, chromosome 13 long arm and chromosome 22 long arm with high frequency.

Using the above results, the chromosome aberrations in primary malignant gliomas and metastatic malignant gliomas were analyzed. The results are shown in Table 4.

**Table 4**

| Primary malignant glioma | | Metastatic malignant glioma | |
|---|---|---|---|
| Amplification | Deletion | Amplification | Deletion |
| 7p | | 8q | |
| | 10 | 10p | 17p |
| | 22q | | |
| 7q | | | |
| 20q | | | |
| 12q | 13q | 12q | 9p |
| | 9p | | 13q |

### Example 3: Analysis of chromosomal aberration in biliary region carcinoma

Using blood and tumor tissues from patients with biliary region carcinoma (15 patients), the same procedure as stated in Example 1 was carried out to prepare chromosome samples and DNA of cancer cells. 15 samples consisted of 5 gallbladder cancer, 1 intrahepatic cholangioma, 2 hepatic portal cholangiomas, 4 extrahepatic cholangiomas and 3 papilloma. Preparation of genomic DNA, labeling, hybridization and detection were carried out by the same method as in Example 1.

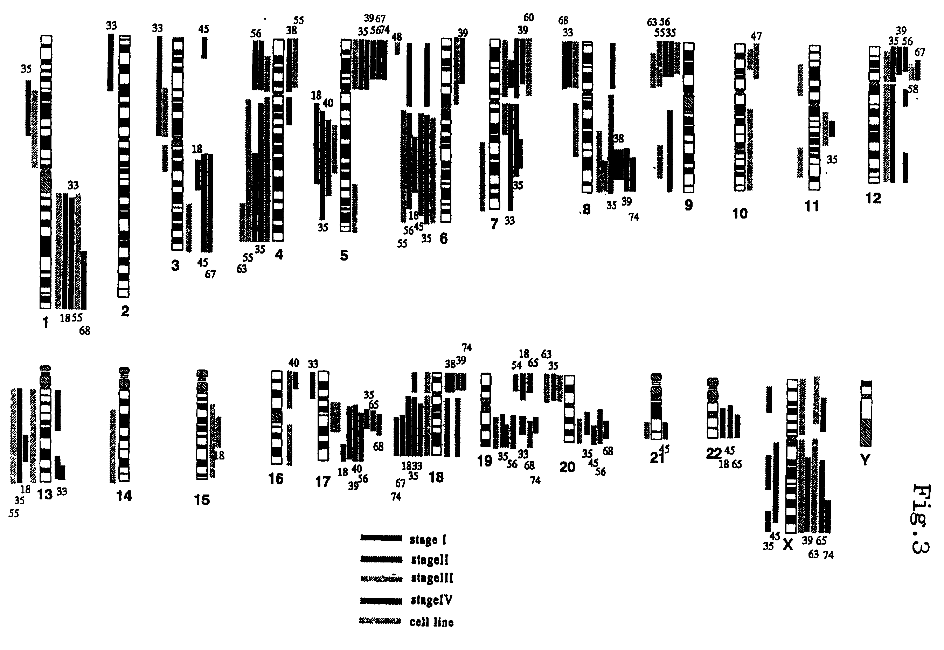

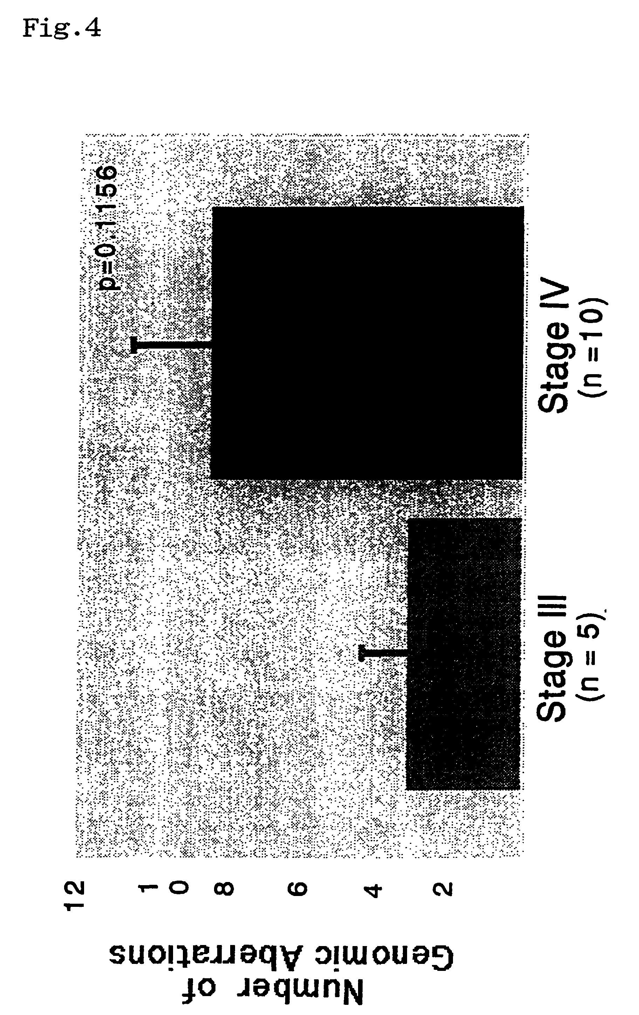

The results are shown in Figure 3 and Tables 5 and 6. In Figure 3, the bars on the right and left of each chromosome respectively represent amplification and deletion in the corresponding chromosome region. The underlined figure represents the patient's number. The number of genomic aberration in each grade of cancer is shown in Figure 4.

**Table 5**

| Chromosome region with amplification | Frequency | Candidate gene |
|---|---|---|
| 17q | 8/19 | c-erbB-2 |
| 19q | 7/19 | |
| 12p | 6/19 | |
| 5p | 6/19 | |
| 8q | 6/19 | c-myc |
| 1q | 5/19 | |
| 7p | 5/19 | EGFR |
| 20q | 5/19 | |
| Xq | 5/19 | |
| 19p | 3/19 | |
| 22q | 3/19 | |
| Xp | 3/19 | |

**Table 6**

| Chromosome region with deletion | Frequency | Candidate gene |
|---|---|---|
| 6q | 6/19 | |
| 18q | 6/19 | DDC, DPC4 |
| 4q | 5/19 | |
| 9p | 5/19 | p16/p15 |
| 5q | 4/19 | APC, MCC |
| 13q | 4/19 | RB |

The above results indicate that amplification in chromosome 17 long arm, chromosome 19 long arm, chromosome 12 short arm, chromosome 5 short arm, chromosome 8 long arm, chromosome 1 long arm, chromosome 7 short arm, chromosome 20 long arm and chromosome X long arm, and deletion in chromosome 6 long arm, chromosome 4 long arm, chromosome 5 long arm, chromosome 9 short arm, chromosome 13 long arm and chromosome 18 long arm were frequently found in biliary region carcinoma,.

More specifically, it is shown that amplification at 17q12-21, 19q13. 1-13. 2, 12p12, 5p13. 2-p ter, 8q24, 1q31-q ter, 7p12-15, 20q13. 1 and Xq25-q ter, and deletion at 6q12-32, 4q, 5q, 9p21-23, 13q, 18q21-q ter were frequently found in biliary region carcinoma,.

Taking note of the total number of chromosome regions wherein aberrations were found, Grade IV tends to be higher than Grade III in number.

Carcinoma of the gallbladder with anomalous confluence of the pancreaticobiliary ductal system showed amplification in chromosome 18 long arm, while carcinoma of the gallbladder without that anomalous confluence showed deletion in the same chromosome.

Analyzing more cases, the relation between the cases of Grades III and IV and amplification or deletion in each chromosome was studied. The results are shown in Table 7.

**Table 7**

| | | Chromosome region observed in 3 or more cases | Chromosome region observed in 2 cases |
|---|---|---|---|
| Grade III | amplification | 7p (7p ter-p13) | 1q (1q21-q ter) |
| | | | 4p (4p ter-p12) |
| | | | 10p (10p ter-p12) |
| | deletion | 4q (4q23-q ter) | |
| | | 6p (6p ter-p23) | |
| | | 6q (6q16-q24) | |
| | | 9p (9p ter-p21) | |
| | | 9q (9q31-q ter) | |
| | | 13q | |
| Grade IV | amplification | 3q (3q24-q ter) | 7q (7q31) |
| | | 5p (5p ter-p14) | 15q (15q21-23) |
| | | 8q (8q24. 1-q ter) | 18q (18q11-q ter) |
| | | 12p (12p12) | |
| | | 17q (17q12-q21) | |
| | | 18p (18p ter-p11.2) | |
| | | 19p (19p ter-p13. 1) | |
| | | 19q (19q13. 1-13. 2) | |
| | | 20q (20q13. 1-q ter) | |
| | | 22q (22q13. 1-q ter) | |
| | | Xq (Xq23-q ter) | |
| | deletion | 5q (5q13-q14) | 4p (4p ter-p12) |
| | | 18q (18q21-q ter) | 17p (17p ter-p11.2) |
| | | | Xq (Xq21-q23) |

### Example 4: Analysis of chromosomal aberration in ovarian clear cell adenocarcinoma

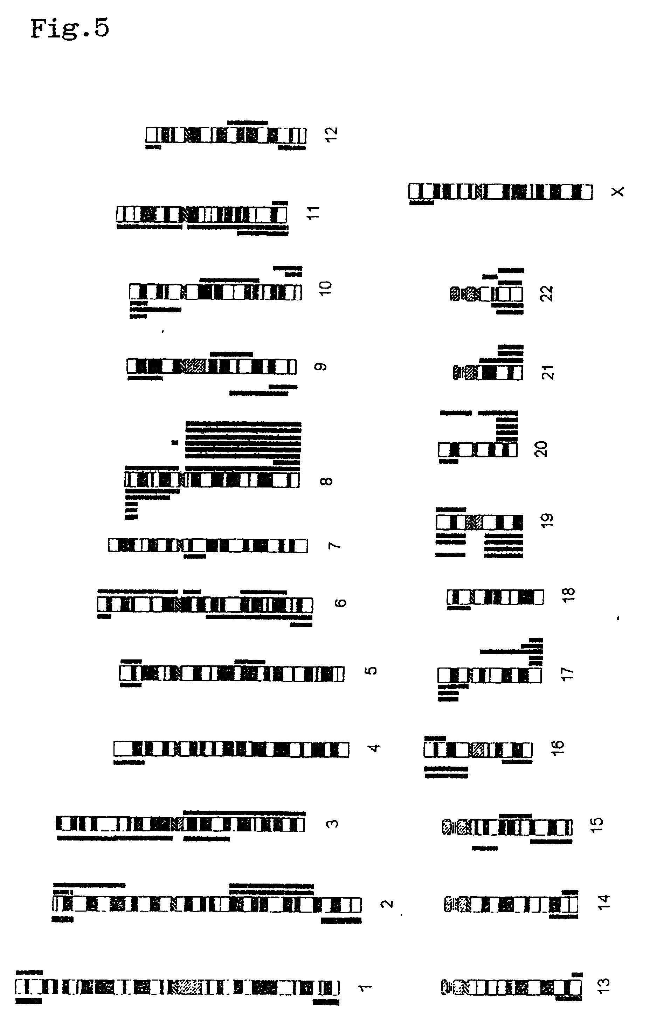

Using blood and tumor tissues from patients with ovarian clear cell adenocarcinoma (12 patients), the same procedure as stated in Example 1 was carried out to prepare chromosome samples and DNA of cancer cells. Classifying 12 samples by clinical progressive grades, 8 samples turned out to be in Grade I, 2 samples in Grade II, 1 sample in Grade III and 1 sample in Grade IV. Preparation of genomic DNA, labeling, hybridization and detection were carried out by the same method as in Example 1. The results are shown in Figure 5 and Table 8. In Figure 5, the bars on the right and left of each chromosome respectively represent amplification and deletion in the corresponding chromosome region.

**Table 8**

| CNA and region | No. | Frequency |
|---|---|---|
| -8p23-p ter | 5 | 42% |
| +8q11-q23 | 7 | 58% |
| +8q24-q ter | 8 | 67% |
| -10p15-p ter | 3 | 25% |
| -17p12-p ter | 3 | 25% |
| +17q25-q ter | 5 | 42% |
| -19p | 3 | 25% |
| -19q | 4 | 33% |
| +20q13-q ter | 5 | 42% |
| +21q22-q ter | 3 | 25% |

The above results indicate that, as a common change of ovarian clear cell adenocarcinoma, there are deletions in chromosome 6 long arm, chromosome 8 short arm, chromosome 10 short arm, chromosome 17 short arm and chromosome 19, and amplifications in chromosome 8 long arm, chromosome17 long arm, chromosome 20 long arm, chromosome 21 long arm and chromosome22 long arm. By comparing this result with the result obtained by the analysis of genetic change of tissular ovarian cancer other than ovarian clear cell adenocarcinoma using CGH method, it has been revealed that deletion in chromosome 10 short arm and amplification in chromosome 17 long arm are characteristic only for clear cell adenocarcinoma. Further, when an examination was carried out in the same manner of this example, amplification in chromosome 3 long arm was found with significantly high frequency in non-clear cell adenocarcinoma.

A comparison of clear cell adenocarcinoma between clinical progressive grades I and II with regard to chromosome region with amplification or deletion did not reveal a significant difference.

A comparison of chromosome regions which have different copy numbers of genes showed a strong correlation in 8q11-21, 17q25-q ter and 20q13-q ter of those chromosome regions.

### Example 5: Analysis of chromosomal aberration in corpus uteri cancer

Using blood and tumor tissues from patients with corpus uteri cancer (14 patients), the same procedure as stated in Example 1 was carried out to prepare chromosome samples and DNA of cancer cells. Preparation of genomic DNA, labeling, hybridization and detection were carried out by the same method as in Example 1.

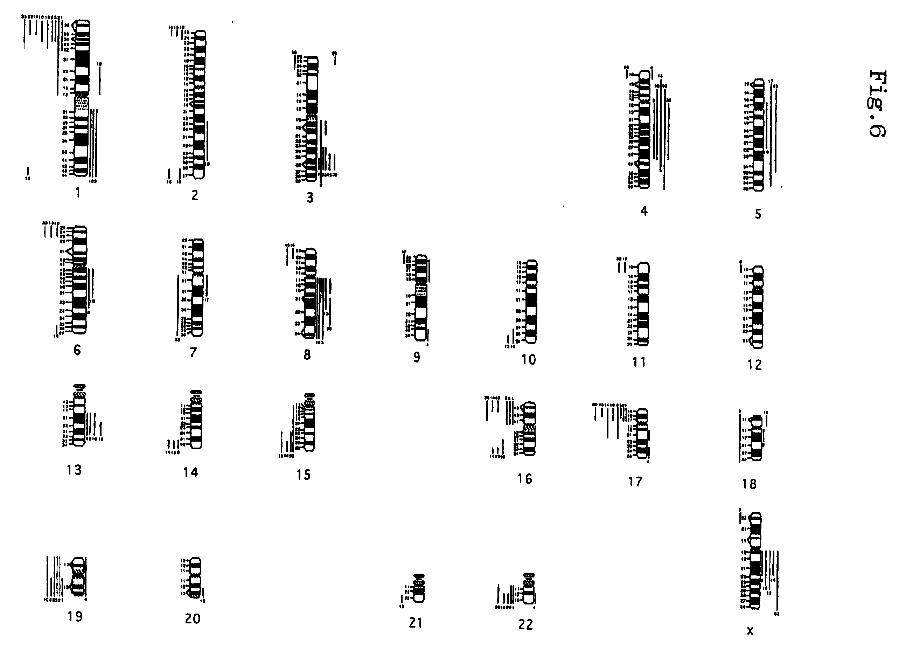

The results are shown in Figure 6 and Table 9. In Figure 6, the bars on the right of each chromosome represent amplification and those on the left represent deletion.

**Table 9**

| Amplification | | Deletion | |
|---|---|---|---|
| Chromosome region | Frequency (n=13) | Chromosome region | Frequency (n=13) |
| 1q | 23.1% | 1p32-33 | 30.8% |
| 3q26 | 46.2% | 1p34-35 | 53.8% |
| 4p12-14 | 23.1% | 1p36-q ter | 69.2% |
| 4q12-26 | 38.5% | 2p25-p ter | 23.1% |
| 5p12-q21 | 23.1% | 6p23-p ter | 23.1% |
| 8q11-13 | 38.5% | 14q32 | 23.1% |
| 8q21 | 38.5% | 15q23-24 | 23.1% |
| 8q22-23 | 30.8% | 15q25-q ter | 30.8% |
| 13q21 | 23.1% | 16p11-12 | 30.8% |
| 13q22-31 | 30.8% | 16p13-p ter | 46.2% |
| Xq12-21 | 38.5% | 16q23-q ter | 23.1% |
| Xq23-24 | 23.1% | 17p11 | 46.2% |
| | | 17p12-p ter | 61.6% |
| | | 19p | 30.8% |
| | | 19q | 46.2% |
| | | 22q11 | 23.1% |
| | | 22q12-q ter | 38.5% |

The above results show that amplification in chromosome 3 long arm and chromosome 8 long arm, and deletion in chromosome 1 short arm, chromosome16 short arm, chromosome 17 short arm and chromosome 19 long arm were found in high frequency. Also, amplification in chromosome 4 long arm, chromosome13 long arm and chromosome X long arm, and deletion in chromosome 15 long arm and chromosome 22 long arm were found in high frequency.

### Example 6: Analysis of chromosomal aberration in hepatocellular carcinoma

Using blood and tumor tissues from patients with hepatocellular carcinoma, the same procedure as stated in Example 1 was carried out to prepare chromosome samples and DNA of cancer cells. Preparation of genomic DNA, labeling, hybridization and detection were carried out by the same method as in Example 1.

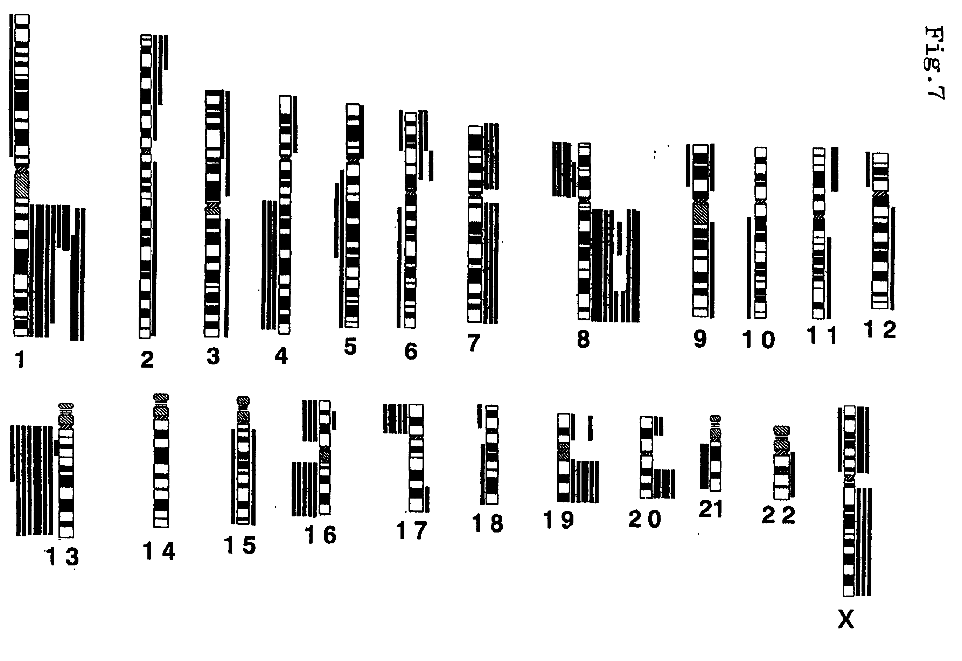

The results are shown in Figure 7. In Figure 7, the bars on the right of each chromosome represent amplification and those on the left represent deletion.

The results of the above analysis show that, in hepatocellular carcinoma, amplification in chromosome 1 long arm, chromosome 8 long arm, chromosome 19 long arm and chromosome 20 long arm, and deletion in chromosome 8 short arm, chromosome13 long arm, chromosome 16 long arm and chromosome 17 short arm were found in high frequency.

More specifically, it shows that, in hepatocellular carcinoma, amplification at 1q22-23, 8q24-q ter, 19q and 20q12-q ter, and deletion at 8p11-21, 13q13-14.1, 16q and 17p were found in high frequency.

Analyzing more cases, the relation between the size of tumor portion and the amplification or deletion of each chromosome was analyzed. The results are shown in Table 10. They indicate that the region with chromosomal aberration increases as the size of tumor portion enlarges.

**Table 10**

| The diameter of tumor portion | Chromosome region with amplification (Frequency) | Chromosome region with deletion (Frequency) |
|---|---|---|
| less than 2cm | 8q24 - q ter (4/5) | 4q12 - q23 (2/5) |
| | 1q23 - q31 (2/5) | 8p (2/5) |
| | | 13q12 - q21.2 (2/5) |
| | | 16q (2/5) |
| | | 17p (2/5) |
| not less than 2cm | 8q24 - q ter (10/13) | 4q12 - q23 (3/13) |
| less than 5cm | 1q23 - q31 (11/13) | 10q (4/13) |
| | 6p22.3 - p25 (3/13) | 13q12 - q21.2 (7/13) |
| | 11q13 - q14.3 (3/13) | 16q (8/13) |
| | Xq (3/13) | 17p (8/13) |
| | | 21q (3/13) |
| not less than 5cm | 8q24 - q ter (4/10) | 4q12 - q23 (4/10) |
| | 1q23 - q31 (6/10) | 8p (3/10) |
| | 6p22.3 - p25 (2/10) | 13q12 - q21.2 (6/10) |
| | 11q13 - q14.3 (3/10) | 16q (4/10) |
| | Xq (2/10) | 17p (3/10) |
| | 2p22 - p ter (2/10) | 21q (2/10) |
| | 7q (2/10) | 1p34.3 - p ter(2/10) |
| | 19q (2/10) | 15q (2/10) |
| | 20q (3/10) | 22q (2/10) |

Furthermore, deletion at 16q was found in 65% of hepatitis C virus-positive hepatoma, and deletion at 10q was found in 30 % of the same disease. This deletion was not found in hepatitis B virus-positive hepatoma. On the other hand, amplification at 11q13 was found in 40% of hepatitis B virus-positive hepatoma, but the same amplification was found in only 5% of hepatitis C virus-positive hepatoma.

### Example 6: Analysis of chromosomal aberration in pituitary adenoma

Using tumor tissues from patients with pituitary adenoma (11 patients), the same procedure as stated in Example 1 was carried out to prepare chromosome samples and DNA of cancer cells. Preparation of genomic DNA, labeling, hybridization and detection were carried out by the same method as in Example 1.

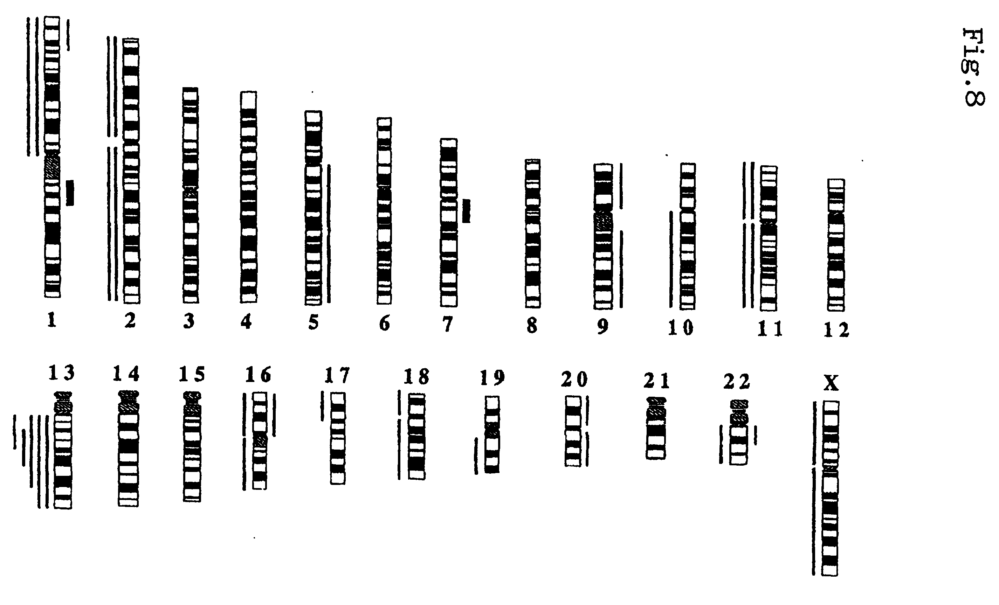

Each chromosome region, which showed green flourescence and exhibited DNA amplification specific for pituitary adenoma, is shown in Figure 8 and Table 11. In Figure 8, the bars on the right of each chromosome represent amplification and those on the left represent deletion. The chromosome region, which showed particularly strong green flourescence and exhibited an increased amount of amplification of DNA therein, is indicated in bold line.

**Table 11**

| Chromosome region with amplification | Frequency |
|---|---|
| 1p (at the edge) | 1/11 |
| 1q (near the center) | 1/11 |
| 5q | 1/11 |
| 7q (near the center) | 1/11 |
| 9p | 1/11 |
| 9q | 1/11 |
| 16p | 1/11 |
| 20p | 1/11 |
| 20q | 1/11 |
| 22q (near the center) | 1/11 |

Each chromosome region, which showed red flourescence and exhibited specific deletion for pituitary adenoma, is shown in Figure 8 and Table 12. In Figure 8, chromosome region with deletion is indicated by a bar on the left of each chromosome.

**Table 12**

| Chromosome region with deletion | Frequency |
|---|---|
| 1p | 2/11 |
| 2p | 2/11 |
| 2q | 2/11 |
| 10q | 1/11 |
| 11p | 2/11 |
| 11q | 2/11 |
| 13q14 | 5/11 |
| 16p | 1/11 |
| 16q | 1/11 |
| 17p | 1/11 |
| 18p | 1/11 |
| 18q | 1/11 |
| 19q | 1/11 |
| 22q | 1/11 |
| Xp | 1/11 |
| Xq | 1/11 |

The above results indicate that deletion in chromosome 13 long arm was found with relatively high frequency in pituitary adenoma.

### Example 7: Analysis of chromosomal aberration in stomach cancer

Using blood and tumor tissues from patients with stomach cancer (33 patients), the same procedure as stated in Example 1 was carried out to prepare chromosome samples and DNA of cancer cells. Preparation of genomic DNA, labeling, hybridization and detection were carried out by the same method as in Example 1.

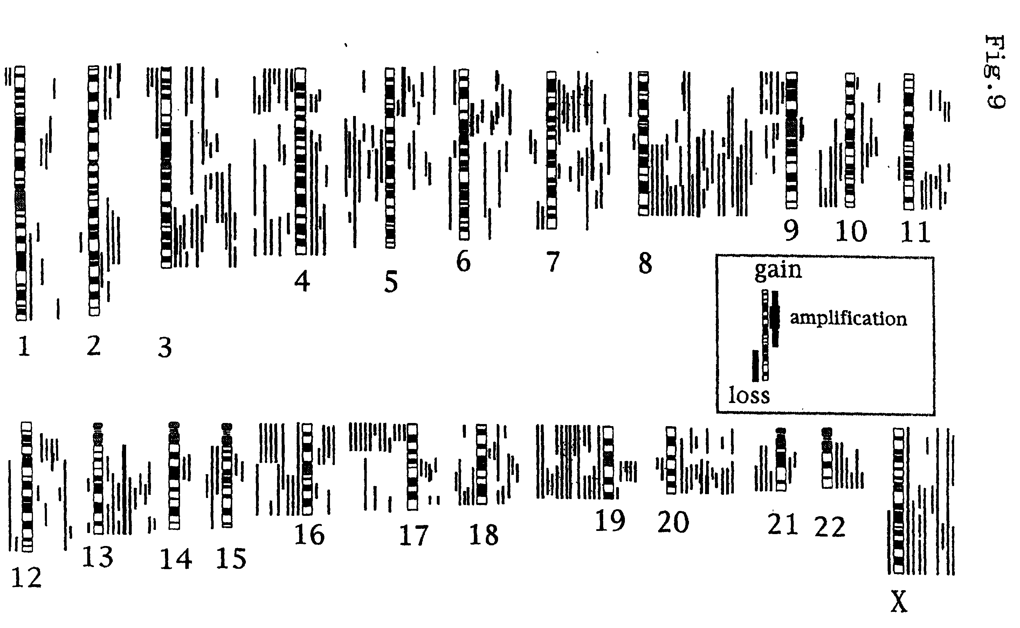

The results are shown in Figure 9, Tables 13 and 14. In Figure 9, the bars on the right of each chromosome represent amplification and those on the left represent deletion. The chromosome region, which showed particularly strong green flourescence and exhibited an increased amount of amplification of DNA therein, is indicated by bold line.

**Table 13**

| Chromosome region with amplification | Frequency |
|---|---|
| 8q23 - ter | 17/33 |
| 3q24 | 11/33 |
| 20q13.1 | 11/33 |
| 3q25.2 - 26.1 | 9/33 |
| 7p cen - 21 | 8/33 |
| 7q22 - 31 | 8/33 |
| 13q21 | 8/33 |
| Xq22.1 - 23 | 8/33 |
| Xq25 - ter | 8/33 |
| 6p cen - 21.2 | 7/33 |
| Xq13 - 21.2 | 7/33 |
| 3q13 | 6/33 |
| 3q27 - ter | 6/33 |
| 13q32 - ter | 6/33 |
| 20p11.2 - 12 | 6/33 |
| Xp21.3 - 22.2 | 6/33 |
| 3p13 | 5/33 |
| 5p14 | 5/33 |
| 5p13 | 5/33 |
| 11q22 - ter | 5/33 |

**Table 14**

| Chromosome region with deletion | Frequency |
|---|---|
| 19q13.2 - ter | 13/33 |
| 17p13 | 11/33 |
| 17p11 | 15/33 |
| 19p | 11/33 |
| 5q14 - 21 | 7/33 |
| 4p16 | 6/33 |
| 4p14 | 6/33 |
| 5q12 - 13 | 6/33 |
| 16p | 6/33 |
| 16q12 - 13 | 6/33 |
| 16q23 - ter | 6/33 |
| 22q13.1 - ter | 6/33 |
| 4q32 | 5/33 |
| 9p13 - 21 | 5/33 |

The above results indicate that amplification in chromosome 8 long arm, chromosome 3 long arm and chromosome 20 long arm, and deletion in chromosome 19 long arm, chromosome 17 short arm and chromosome 19 short arm were frequently found in stomach cancer.

More specifically, amplifications at 8q23 ― ter, 3q24 and 20q13.1 were frequently found in stomach cancer, and deletions at 19q13.2 ― ter, 17p13, 17p11 and 19p were frequently found in stomach cancer.

Besides the above, amplification was seen in chromosome regions listed in Table 13, and deletion was seen in chromosome regions listed in Table 14. Each chromosome region listed in Table 15 represents the part where particularly strong green flourescence appeared, and accordingly it exhibits an increased amount of amplification of DNA therein.

**Table 15**

| Chromosome region with increased amount of amplification | Frequency |
|---|---|
| 2p24 - ter | 1/33 |
| 3q25.2 - 27 | 1/33 |
| 5p | 1/33 |
| 6p24 | 1/33 |
| 6p12 - 21.3 | 3/33 |
| 7p13 - 21 | 2/33 |
| 8p22 | 1/33 |
| 8q13 - ter | 1/33 |
| 9q12 | 1/33 |
| 12pcen - 12 | 2/33 |
| 13q | 1/33 |
| 17q21 | 1/33 |
| 19q cen - 13.1 | 2/33 |
| 20q13.1 - ter | 2/33 |

### Example 8: Analysis of chromosomal aberration in esophageal squamous cell carcinoma

Using blood and tumor tissues from patients with esophageal squamous cell carcinoma (18 patients), the same procedure as stated in Example 1 was carried out to prepare chromosome samples and DNA of cancer cells. Preparation of genomic DNA, labeling, hybridization and detection were carried out by the same method as in Example 1.

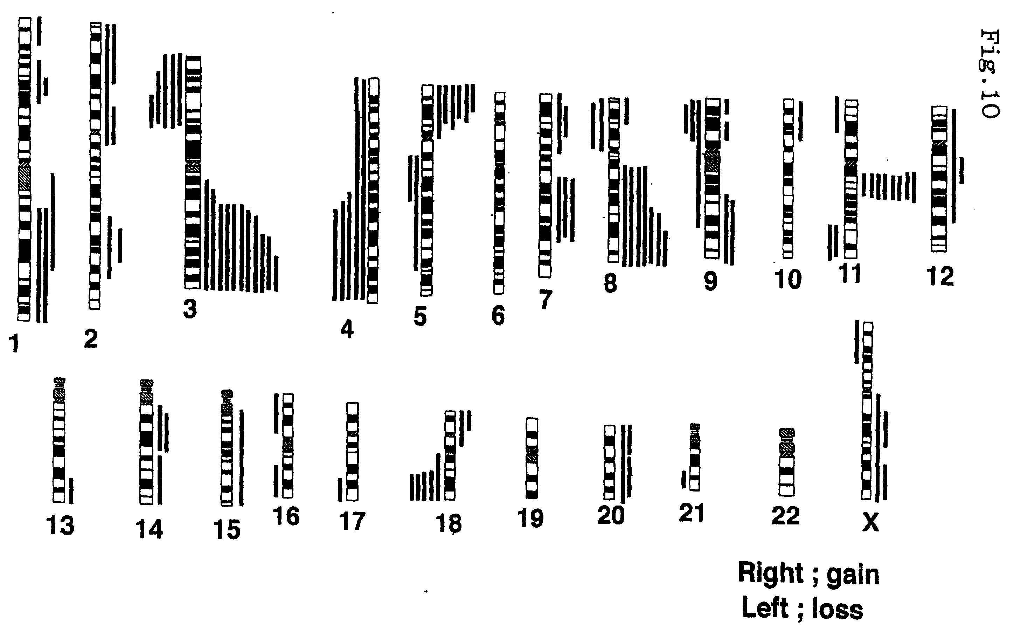

The results are shown in Figure 10 and Table 16. In Figure 10, the bars on the right of each chromosome represent amplification and those on the left represent deletion.

**Table 16**

| Amplification | | Deletion | |
|---|---|---|---|
| Chromosome region | Frequency | Chromosome region | Frequency |
| 3q26.2 - q ter | 61% | 3p14.2 - 21.3 | 28% |
| 3q24 - 26.1 | 56% | 4q26 - q ter | 28% |
| 3q21 - 23 | 50% | 18q21 - q ter | 28% |
| 11q13 | 44% | 3p22 - 21.1 | 22% |
| 8q23 - q ter | 39% | 3p24.2 - p ter | 15% |
| 8q22 | 33% | 9p21 - 23 | 15% |
| 5p14 - p ter | 33% | | |
| 8q12 - 21.3 | 22% | | |

The above results indicate that amplification in chromosome 3 long arm, chromosome 11 long arm, chromosome 8 long arm and chromosome 5 short arm was frequently found in esophageal squamous cell carcinoma.

More specifically, it is showed that amplifications at 3q26.2 ― q ter, 3q24 ― 26.1, 3q21 ― 23, 11q13, 8q22 ― q ter, 5p14 ― p ter and 8q12 ― 21.3 were frequently found in esophageal squamous cell carcinoma. At the same time, there were some cases with deletion in chromosome 3 short arm, chromosome 4 long arm, chromosome 18 long arm and chromosome 9 short arm. To be more specific, there were the cases with deletion at 3p14.2 ― 21.3, 4q26 ― q ter, 18q21 ― q ter, 3p22 ― 21.1, 3p24.2 ― p ter and 9p21 ― 23.

### Example 9: Analysis of chromosomal aberration in oral squamous cell carcinoma

Using cancer cells from patients with oral squamous cell carcinoma (14 cell strains), the same procedure as stated in Example 1 was carried out to prepare chromosome samples and DNA of cancer cells. Preparation of genomic DNA, labeling, hybridization and detection were carried out by the same method as in Example 1.

The results are shown in Table 17.

**Table 17**

| Amplification | | Deletion | |
|---|---|---|---|
| Chromosome region | Frequency | Chromosome region | Frequency |
| 5p | 85.7% | 18q | 71.4% |
| 8q22 - ter. | 78.5% | 4q31 - ter. | 64.3% |
| 20p | 57.1% | 1q21 - 23. | 42.9% |
| 20q | 57.1% | 4p | 28.6% |
| 3q25 - ter. | 50% | 21q | 21.4% |
| Xq21 | 35.7% | 16p | 14.8% |
| 2q22 - 32 | 28.6% | 19p | 14.8% |
| 11p | 28.6% | 19q | 14.8% |
| 7p | 21.4% | 22q | 14.8% |
| 1p21 - 31 | 14.3% | | |
| 5q23 - 34 | 14.3% | | |
| 6q22.3 - 25.1 | 14.8% | | |
| 9q33 - ter. | 14.8% | | |
| 12q22 - 23. | 14.8% | | |

The above results indicate that amplifications in chromosome 5 short arm, chromosome 8 long arm, chromosome 20 short arm, chromosome 20 long arm and chromosome 3 long arm were frequently found in oral squamous cell carcinoma, and deletions in chromosome 18 long arm and chromosome 4 long arm were frequently found in oral squamous cell carcinoma.

More specifically, it is showed that amplifications at 5p, 8q22 ― ter, 20p, 20q and 3q25 ― ter, and deletion at 18q and 4q31 ― ter were frequently found in oral squamous cell carcinoma.

Besides the above specified, amplification was found at Xq21, 2q22 ― 32, 11p, 7p, 1p21 ― 31, 5q23 ― 34, 6q22.3 ― 25.1, 9q33 ― ter, 12q22 ― 23, and deletion was found at 1q21 ― 23, 4p, 21q, 16p, 19p, 19q and 22q.

### Industrial Applicability

According to the above stated test, it has now been revealed that, regarding meningioma, glioma, pituitary adenoma, biliary region carcinoma, ovarian clear cell adenocarcinoma, non-clear cell adenocarcinoma, corpus uteri cancer, hepatocellular carcinoma, stomach cancer, esophageal squamous cell carcinoma and oral squamous cell carcinoma, the amplification or deletion of DNA occurs in the chromosome regions specific for each cancer cell of the above cancers. By applying the present invention which utilizes the relation between the structural change of such chromosomes and the onset of a cancer, it is possible to diagnose the tumor of patients properly. Furthermore, according to the method of the present invention, it is possible to identify a chromosome region which is involved in a specific tumor, and so the invention is also applicable to identify a gene which is involved in the carcinogenesis and so on.

## Claims

1. A method for diagnosing benign meningioma which comprises a step of detecting deletion in human chromosome 22 long arm in the chromosomes of cancer cells derived from a patient.

2. A method for diagnosing atypical meningioma which comprises a step of detecting deletion in human chromosome 1 short arm, human chromosome 6 long arm, human chromosome 14 long arm, human chromosome 18, or human chromosome 22 long arm in the chromosomes of cancer cells derived from a patient.

3. A method for diagnosing anaplastic meningioma which comprises a step of detecting amplification in human chromosome 20 long arm, or detecting deletion in human chromosome 1 short arm, human chromosome 2 short arm, human chromosome 6 long arm, human chromosome 10, human chromosome 14 long arm or human chromosome 22 long arm, in the chromosomes of cancer cells derived from a patient.

4. A method for diagnosing malignant glioma which comprises a step of detecting amplification in human chromosome 7 short arm, human chromosome 7 long arm, human chromosome 20 long arm or human chromosome 8 long arm, or detecting deletion in human chromosome 10 short arm, human chromosome 9 short arm, human chromosome 10 long arm, human chromosome 13 long arm or human chromosome 22 long arm in the chromosomes of cancer cells derived from a patient.

5. A method for diagnosing primary malignant glioma which comprises a step of detecting amplification in human chromosome 7 short arm, human chromosome 7 long arm or human chromosome 20 long arm, or detecting deletion in human chromosome 10 or human chromosome 22 long arm in the chromosomes of cancer cells derived from a patient.

6. A method for diagnosing metastatic malignant glioma which comprises a step of detecting amplification in human chromosome 8 long arm or human chromosome 10 short arm, or detecting deletion in human chromosome 17 short arm in the chromosomes of cancer cells derived from a patient.

7. A method for diagnosing biliary region carcinoma which comprises a step of detecting amplification in human chromosome 17 long arm, human chromosome 19 long arm, human chromosome 12 short arm, human chromosome 5 short arm, human chromosome 8 long arm, human chromosome 1 long arm, human chromosome 7 short arm, human chromosome 20 long arm or human chromosome X long arm, or detecting deletion in human chromosome 6 long arm, human chromosome 4 long arm, human chromosome 5 long arm, human chromosome 9 short arm, human chromosome 13 long arm or human chromosome 18 long arm in the chromosomes of cancer cells derived from a patient.

8. A method for diagnosing anomalous confluence of the pancreaticobiliary ductal system in gallbladder cancer which comprises a step of detecting amplification or deletion in human chromosome 18 long arm in the chromosomes of cancer cells derived from a patient.

9. A method for diagnosing ovarian clear cell adenocarcinoma which comprises a step of detecting amplification in human chromosome 8 long arm, human chromosome 17 long arm, human chromosome 20 long arm, human chromosome 21 long arm or human chromosome 22 long arm, or detecting deletion in human chromosome 6 long arm, human chromosome 8 short arm, human chromosome 10 short arm, human chromosome 17 short arm, or human chromosome 19 in the chromosomes of cancer cells derived from a patient.

10. A method for diagnosing non-clear cell adenocarcinoma which comprises a step of detecting amplification in human chromosome 3 long arm in the chromosomes of cancer cells derived from a patient.

11. A method for diagnosing corpus uteri cancer which comprises a step of detecting amplification in human chromosome 3 long arm, human chromosome 8 long arm, human chromosome 4 long arm, human chromosome 13 long arm or human chromosome X long arm, or detecting deletion in human chromosome 1 short arm, human chromosome 16 short arm, human chromosome 17 short arm, human chromosome 19 long arm, human chromosome 15 long arm or human chromosome 22 long arm in the chromosomes of cancer cells derived from a patient.

12. A method for diagnosing hepatocellular carcinoma which comprises a step of detecting amplification in human chromosome 1 long arm, human chromosome 8 long arm, human chromosome 19 long arm or human chromosome 20 long arm, or detecting deletion in human chromosome 8 short arm, human chromosome 13 long arm, human chromosome 16 long arm or human chromosome 17 short arm in the chromosomes of cancer cells derived from a patient.

13. A method for diagnosing hepatitis C virus-positive hepatoma which comprises a step of detecting deletion in human chromosome 16 long arm or human chromosome 10 long arm in the chromosomes of cancer cells derived from a patient.

14. A method for diagnosing hepatitis B virus-positive hepatoma which comprises a step of detecting amplification at human chromosome 11 long arm 13, in the chromosomes of cancer cells derived from a patient.

15. A method for diagnosing pituitary adenoma which comprises a step of detecting deletion in human chromosome 13 long arm in the chromosomes of cancer cells derived from a patient.

16. A method for diagnosing stomach cancer which comprises a step of detecting amplification in human chromosome 8 long arm, human chromosome 3 long arm or human chromosome 20 long arm, or detecting deletion in human chromosome 17 short arm, human chromosome 19 short arm or human chromosome 19 long arm in the chromosomes of cancer cells derived from a patient.

17. The method for diagnosing stomach cancer of claim 16 which comprises a step of detecting amplification at human chromosome 20 long arm 13.1.

18. A method for diagnosing esophageal squamous cell carcinoma which comprises a step of detecting amplification in human chromosome 3 long arm, human chromosome 11 long arm, human chromosome 8 long arm or human chromosome 5 short arm, or detecting deletion in human chromosome 3 short arm, human chromosome 4 long arm, human chromosome 9 short arm or human chromosome 18 long arm in the chromosomes of cancer cells derived from a patient.

19. A method for diagnosing oral squamous cell carcinoma which comprises a step of detecting amplification in human chromosome 5 short arm, human chromosome 8 long arm, human chromosome 20 short arm, human chromosome 20 long arm or human chromosome 3 long arm, or detecting deletion in human chromosome 18 long arm or human chromosome 4 long arm in the chromosomes of cancer cells derived from a patient.

20. The method of diagnosis of any one of claims 1-19 wherein said amplification or deletion is detected by:
competitively hybridizing DNA of cancer cells derived from a patient which is labeled with a first label and DNA of normal cells which is labeled with a second label, to chromosomes of normal cells; and
observing a chromosome region to which DNA of cancer cells is hybridized by the first label, observing a chromosome region to which DNA of normal cells is hybridized by the second label, observing a chromosome region to which both DNAs are hybridized by a mixture of the first and second labels, and identifying the labels in each region of the chromosome.

21. The method of diagnosis of any one of claims 1-19 wherein said amplification or deletion is detected by:
competitively hybridizing DNA of cancer cells derived from a patient which is labeled to be detected as a first color and DNA of normal cells which is labeled to be detected as a second color, to chromosomes of normal cells; and
observing a chromosome region to which DNA of cancer cells is hybridized by the first color, observing a chromosome region to which DNA of normal cells is hybridized by the second color, observing a chromosome region to which both DNAs are hybridized by the third color formed by a mixture of the first and second colors, and identifying the colors in each region of the chromosome.

22. The method of diagnosis of claim 21 wherein said amplification or deletion is detected by measuring the ratio of intensity between the first and second colors in each region of the chromosome.
